# EUROPEAN PATENT SPECIFICATION

(11) **EP 1 622 843 B1**
(45) Date of publication and mention of the grant of the patent: **03.06.2015**
(21) Application number: 04719777.7
(22) Date of filing: 11.03.2004
(51) Int. Cl.: C04B 12/02, A61L 24/00, A61K 6/06, C04B 24/04, C04B 28/34

(54) **PREMIXED SELF-HARDENING BONE GRAFT PASTES**
VORGEMISCHTE SELBSTHÄRTENDE KNOCHENTRANSPLANTATPASTEN
PATES POUR GREFFE OSSEUSE AUTODURCISSANTES PREMELANGEES

(30) Priority: 08.04.2003 US 461338 P
(43) Date of publication of application: 08.02.2006
(73) Proprietor: ADA FOUNDATION, Chicago, IL 60611-2678 (US)
(72) Inventor: CHOW, Laurence, C., Germantown, Maryland 20874 (US); TAKAGI, Shozo, Gaithersburg, Maryland 20878 (US)
(74) Representative: Hoffmann, Benjamin
(86) International application number: PCT/US2004/007422
(87) International publication number: WO 2004/093734

(56) References cited:
- EP-A- 0 520 690
- EP-A- 0 538 913
- EP-A- 0 639 366
- JP-A- 2 311 406
- JP-A- 2001 170 161
- US-A- 4 902 649
- US-A1- 2002 137 812

## Description

### BACKGROUND OF THE INVENTION

The various embodiments of the present invention are generally directed to self-hardening calcium phosphate-containing and/or calcium-containing cement compositions. The compositions may be used to form pastes for bone and tooth restoration and similar applications, where the paste will harden within a desired time after being delivered to a repair site.

Most conventional calcium phosphate cements are mixed with an aqueous solution immediately before application. In the clinical situation, the ability of the surgeon to properly mix the cement and then place the cement paste in the defect within the prescribed time is a crucial factor in achieving optimum results.

A self-hardening calcium phosphate cement ("CPC"), consisting of tetracalcium phosphate (Ca₄(PO₄)₂O, also referred to as "TTCP") and dicalcium phosphate anhydrous (CaHPO₄, also referred to as "DCPA"), has been shown in clinical studies to be efficacious for repairing bone defects. The hardening time of such conventional cements is as long as about 30 minutes with water, although hardening time can be shortened if a phosphate solution is used as the cement liquid. Hydroxyapatite (Ca₅(PO₄)₃OH, also referred to as "HA") is formed as the product. More recently, additional CPCs that do not contain TTCP, e.g., α-tricalcium phosphate (α-Ca₃(PO₄)₂, also referred to as "α-TCP") and CaCO₃ or DCPA and Ca(OH)₂, have also been developed. These cements may harden in about 10 minutes when a phosphate solution is used as the cement liquid. They also form hydroxyapatite ("HA") as the final product.

A premixed CPC paste containing the TTCP and DCPA powders and glycerol as the cement liquid has been used for root canal filling and sealing by injection techniques. The cement paste was found to be stable in a syringe but hardened only after being delivered into the root canal where it became exposed to water from the surrounding tissues. Because the cement paste was injected into a confined area, there was little concern of disintegration of the paste due to washout. Although the premixed CPC was shown to have improved biocompatibility with periapical bone tissue than a number of conventional root canal filling or sealing materials, the premixed CPC-glycerol paste did not exhibit a good washout resistance when it was applied to an open wet field.

Document JP 02 311406 A discloses a water-free composition for dental or bone restoration comprising a non-toxic, non-aqueous, water-miscible liquid (e.g. glycerol), a powdered calcium phosphate (e.g. tricalcium or tetracalcium phosphate) and an organic acid (e.g. citric acid). This document also discloses a paste for bone and tooth restoration and a method of preparing a paste for bone and tooth restoration.

There remains a need for premixed cement pastes that are stable in the package, are resistant to washout, and will harden only after being deposited at the site of the defect but, once placed, will then harden within a predetermined time.

### BRIEF SUMMARY OF THE INVENTION

The various embodiments of the present invention comprise compositions and means for formulating premixed calcium and/or calcium phosphate and organic acid cement pastes that are stable in a package, resistant to washout, and harden within a desired time after being delivered to the defect or implant site. A non-toxic, non-aqueous, water-miscible liquid such as gycerol is preferred as the liquid in the premix because the cement paste hardening reaction to form calcium complexes and HA does not occur in a water-free environment. A carboxylic acid is used to accelerate cement hardening upon delivery to a desired repair site. A gelling agent is also added to improve the paste cohesiveness.

Methods of repairing and restoring bone and tooth tissue include delivering the pastes to the defect site by any suitable methods known to those of skill in the art. The pastes are exposed to an aqueous fluid to promote hardening of the paste to a cement at a relatively rapid rate.

When premixed self-hardening cements are formulated with sodium phosphate ("Na₂HPO₄") to accelerate cement hardening and prepared by mixing glycerol, Na₂HPO₄, and hydroxypropyl methyl cellulose ("HMC") with CPC powders, the cements will harden only after being delivered to a desired site. Although Na₂HPO₄ may serve to accelerate cement hardening, the hardening times ("HT") of these cements can be 60 minutes or longer. Where shorter hardening times are desired, the present compositions include carboxylic acids to accelerate cement hardening provide self-hardening calcium phosphate-containing and/or calcium-containing cement pastes having hardening times of about 35 minutes or less.

Thus, it is an object of the invention to provide a premixed composition of a calcium phosphate-containing and/or calcium-containing cement material which exhibits resistance to washout as well as desirable hardening times.

A further object of the invention is to provide an essentially water-free, cement-forming paste capable of forming calcium complexes and HA after exposure to water for repair of dental material and bone.

Another object of the invention is to provide a method for controlling the hardening times of HA forming cements pastes.

Another object of the invention is to provide an HA forming cement formulation capable of remaining in an injectable paste form until exposed to an aqueous environment.

These and other objects, advantages and features of the invention are set forth in the detailed description which follows.

### DETAILED DESCRIPTION OF THE INVENTION

Premixed calcium cement pastes for use in bone graft and similar medical repair applications are provided. The pastes may be injectable for delivery to the bone or tooth defect site. The pastes include a non-toxic, calcium-containing and/or calcium phosphate-containing powder, a non-toxic carboxylic acid capable of forming calcium complexes, and a non-toxic, non-aqueous, water-miscible liquid. Non-aqueous liquids are preferred to limit premature hardening of the pastes, which may harden in aqueous environments. A preferred liquid is glycerin (also sometimes referred to as "glycerol"). The carboxylic acid is used to accelerate the hardening time of the paste upon delivery. Gelling agents, such as HMC, carboxymethyl cellulose ("CMC"), alginate or chitosan are also mixed with the powders to enhance paste cohesiveness and washout resistance.

Because the hardening of these cements results from calcium-complex formation, it is contemplated that self-hardening cements can also be formulated using calcium-containing compounds instead of, or in combination with, calcium phosphate compounds. The calcium phosphate and/or calcium-containing compound powder can include monocalcium phosphate monohydrate ("MCPM"), monocalcium phosphate anhydrous ("MCPA"), dicalcium phosphate anhydrous ("DCPA"), dicalcium phosphate dehydrate ("DCPD"), octacalcium phosphate ("OCP"), α-TCP, β-tricalcium phosphate ("β-TCP"), amorphous calcium phosphate ("ACP"), calcium deficient HA, non-stoichiometric HA, TTCP, CaSO₄, CaSO₄•0.5 H₂O, CaSO₄•2 H₂O, CaO, Ca(OH)₂, and CaCO₃ and combinations thereof Preferred calcium phosphate powders include TTCP, DCPA, α-TCP and β-TCP. The Ca/P molar ratio of TTCP is preferably between 1.67 to 2, of α-TCP is between 1.5 to 1.67, and of β-TCP is between 1.50 to 1.67. The particle sizes of the calcium phosphate and/or calcium-containing compounds are between 1 to 50 µm.

Any suitable, non-toxic, non-aqueous, water-miscible liquid may be used in preparing the pastes. Possible liquids include glycerin, as well as related liquids, such as glycerin compounds, derivatives, substitutes that are non-toxic, non-aqueous, and water-miscible. Certain alcohols also may be suitable for use as the non-toxic, non-aqueous, water-miscible liquid. Preferably, the liquid is selected from glycerin, propylene glycol, poly(propylene glycol), poly(ethylene glycol), and combinations thereof.

Non-toxic, organic carboxylic acids are used. A number of carboxylic acids form calcium complexes that are not highly soluble. These acids include glycolic, citric, tartaric, malonic, malic, and maleic acids. Some of these acids, when mixed with a powder containing one or more of calcium phosphate compounds and/or calcium-containing compounds produce relatively fast hardening cements. Thus, it is possible that the use of these acids can produce faster setting premixed cements. One or more of these acids are mixed with the powder to provide a stable paste that will harden only upon contact with an aqueous fluid. Without wishing to be bound by any theories, it is believed that the calcium phosphate compounds and/or calcium-containing compounds react with the organic acids in the presence of water to initially form calcium complexes that are not highly soluble, rather than to directly form hydroxyapatite. This then results in more rapid hardening of the paste.

The compositions also include a non-toxic gelling agent to enhance paste cohesiveness and washout resistance. The gelling agent may include HMC, CMC, chitosan, collagen, gum, gelatin, and alginate, and combinations thereof.

The compositions are prepared and stored under substantially anhydrous conditions to limit premature hardening of the cement pastes. The compositions may be employed as self-hardening cement pastes in a variety of medical and dental procedures for repairing or restoring missing or defective bone or tooth tissue. The cement pastes may be applied to the defect site using any suitable methods, including injecting with a syringe or depositing with a spatula, and also molded or sculpted *in vivo* as desired. When the cement pastes are exposed to physiologic fluids, which contain water, or another aqueous environment at the defect site, they will harden relatively rapidly. An aqueous fluid may be contacted with the compositions either prior to or after application of the cement pastes at the defect site to enhance the rate of hardening of the cement pastes. As an example, a sodium phosphate or saline solution may be sprayed over the surface of the cement paste after it is delivered to the defect site to promote hardening of the outer surface of the cement paste, which will also assist with maintaining the shape of the cement paste as applied and molded. As another example, water may be mixed with the cement pastes prior to application of the pastes at the defect site to initiate hardening.

For most clinical applications, a cement hardening time of more than 60 minutes is too long. Premixed pastes or self-hardening bone graft pastes ("BGPs") in accordance with the various embodiments of the present invention will have an HT of no more than about 35 minutes, preferably no more than 20 minutes and even more preferably between about 5 to about 15 minutes.

### EXAMPLES

The following examples further illustrate preferred embodiments of the present invention but are not be construed as in any way limiting the scope of the present invention as set forth in the appended claims.

Various premixed self-hardening pastes were prepared. Hardening times and other properties of the pastes were evaluated.

**Preparation of the solid ingredients of premixed paste:** TTCP was prepared by heating an equimolar mixture of commercially obtained DCPA (Baker Analytical Reagents, J.T. Baker Chemical Co., Phillipsburg, NJ) and CaCO₃ (J.T. Baker Chemical Co.) at 1500°C for 6 hours in a furnace and quenched at room temperature. The TTCP and DCPA powders of the paste compositions were ground individually in a planetary ball mill in cyclohexane, ethanol, or without a liquid to obtain the desired median particle sizes, which typically is about 15 µm as disclosed in the prior art for making CPC powders. The median particle sizes of TTCP and DCPA were about 17.1 µm and about 1.7 µm, respectively.

α-TCP was prepared by heating a mixture that contained 2 mol of DCPA and 1 mol of CaCO₃ to 1500°C for 6 hours and then quenched in air. The powders were ground individually in a planetary ball mill in cyclohexane, ethanol, or without a liquid to obtain the desired median particle sizes based on data from previous studies. The median particle sizes of α-TCP and CaCO₃ were 4.6 µm and 3.9 µm, respectively. The median particle size of Ca(OH)₂ was 2.2 µm. The particle sizes of the components of the pastes prepared in accordance with the present invention is in the range of 1 to 50 µm.

**Liquid ingredients of controls and premixed pastes:** All ingredients were obtained commercially. A homogeneous mixture of a carboxylic acid, HMC or CMC, and glycerin was produced by blending the mixture in a ball mill.

**Preparation of premixed pastes:** Premixed paste compositions were prepared by mixing a powder and a liquid at desired powder-to-liquid mass ratios (P/L) on a mixing block until a smooth and homogenous paste was obtained. The compositions, with components expressed in mass fraction (%) are presented in Table 1.

**Table 1**

| **Paste** | **Solid** | **Liquid** | | | **P**/**L** |
|---|---|---|---|---|---|
| | | **Glycerin** | **Carboxylic Acid** | **Gelling Agent** | |
| P1 | TTCP (73%) | 62.2% | d-tartaric acid (37.5%) | HMC (0.3%) | 3.0 |
| | DCPA (27%) | | | | |
| P2 | TTCP (73%) | 62.2% | glycolic acid (37.5%) | HMC (0.3%) | 3.0 |
| | DCPA (27%) | | | | |
| P3 | TTCP (73%) | 70.5% | malonic acid (29%) | HMC (0.5%) | 3.0 |
| | DCPA (27%) | | | | |
| P4 | TTCP (73%) | 79.5% | maleic acid (20%) | HMC (0.5%) | 3.0 |
| | DCPA (27%) | | | | |
| P5 | TTCP (73%) | 49.3% | citric acid (49.2%) | CMC (1.5%) | 2.3 |
| | DCPA (27%) | | | | |
| P6 | TTCP (39.1%) | 61.9% | d-tataric acid (37.1%) | CMC (1%) | 1.5 |
| | α-TCP (60.9 %) | | | | |
| P7 | TTCP (55%) | 61.9% | d-tataric acid (37.1%) | CMC (1%) | 1.5 |
| | DCPA (20%) | | | | |
| | α-TCP (25%) | | | | |
| P8 | TTCP | 61.9% | d-tataric acid (37.1%) | CMC (1%) | 1.5 |
| P9 | α-TCP | 61.9% | d-tataric acid (37.1%) | CMC (1%) | 1.5 |

**Washout resistance test:** The washout resistance of the premixed pastes was tested as follows. Premixed paste samples were shaped into a small sphere by hand, and then placed immediately in a 5 mL of physiologic-like solution ("PLS") (1.15 mM Ca, 1.2 mM P, 133 mM NaCl, 50 mM HEPES, pH = 7.4) at 37 °C. The sample was considered to pass the washout resistance test if it did not visibly disintegrate in the PLS. All samples exhibited excellent washout resistance.

**Diametral tensile strength ("DTS") measurement:** DTS samples were prepared by placing the premixed paste into a mold (6 mm diameter by 3 mm height) with about 2 MPa of applied pressure. The mold was covered with two fritted glass slides (pore size of about 40 µm to about 60 µm, thickness of about 3.5 mm) and immersed in PLS at 37 °C. Glycerol-PLS exchange occurred through the fritted glass, allowing the paste to harden. Samples were removed from molds at about 4 hours, and then each sample was immersed in 30 mL of PLS for an additional 20 hours. In some cases, additional samples were prepared and samples were immersed in PLS for an additional 6 days with the PLS being changed daily (30 mL/specimen at 37 °C) to investigate the effect of PLS immersion on physicochemical properties. DTS values (standard uncertainty equals 5 %) were measured on a Universal Testing Machine (United Calibration Corp, Garden Grove, CA) using a loading rate of 10 mm/min,

**Hardening time measurements:** The Gilmore needle method (standard uncertainty equals 5 %) was used to measure hardening time on samples prepared as described above for DTS measurements. All samples exhibited short hardening times. The hardening times were as shown in Table 2.

**Table 2**

| Premixed Paste | HT (minutes, mean±standard deviation, n = 3) |
|---|---|
| P1 | 10 ± 1 |
| P2 | 15 ± 1 |
| P3 | 20 ± 1 |
| P4 | 20 ± 1 |
| P5 | 35 ± 1 |
| P6 | 15 ± 1 |
| P7 | 25 ± 1 |
| P8 | 35 ± 1 |
| P9 | 20 ± 1 |

**Assessments of hydroxyapalite formation:** Powder X-ray diffraction ("XRD") analysis was used to estimate the extent of paste conversion to HA. The estimated standard uncertainty in 2θ measurements is 0.01° and the minimum mass fraction of a calcium phosphate phase that can be detected by XRD is about 3 %.

### Diametral Tensile (DTS) Strength

DTS of some of the premixed paste samples were determined as given in Table 3.

**Table 3**

| Paste | 1-day DTS (MPa) | 7-day DTS (MPa) |
|---|---|---|
| P1 | 4.3 ± 0.3 (n =5) | 3.8 ± 0.3 |
| P2 | 3.1 ± 0.5 | 3.0 ± 0.3 |
| P3 | 2.3 ± 0.4 | 2.7 ± 0.3 |

**Hydroxyapatite** ("**HA**") **Formation:** Conversion of the initial cement compositions to HA was incomplete in 1-day samples. Complete and near complete conversion of the initial cement compositions to HA was found in all 7-day samples of premixed pastes using XRD. In sum, formation of a bone replacement or dental replacement paste results by combining dry powder constituents, characterized by their conversion to calcium complexes in the presence of carboxylic acids and water. A gelling agent, such as hydroxypropyl methyl cellulose, is mixed with the powder to improve the cohesiveness of the paste. The ratio of combined constituents is broad and the resulting paste can be formulated to control rather precisely the physical properties of the paste, including injectability, porosity and hardening time.

While particular embodiments of the present invention have been described and illustrated, it should be understood that the invention is not limited thereto as modifications may be made by persons skilled in the art.

## Claims

1. An essentially water-free premixed composition of matter for dental restoration and bone implants and restoration comprising, in combination:
a non-toxic non-aqueous water-miscible liquid;
a powdered calcium compound selected from the group consisting of monocalcium phosphate monohydrate, monocalcium phosphate anhydrous, dicalcium phosphate anhydrous, dicalcium phosphate dehydrate, octacalcium phosphate, α-tricalcium phosphate, β-tricalcium phosphate, amorphous calcium phosphate, calcium deficient hydroxyapatite, non-stoichiometric hydroxyapatite, tetracalcium phosphate, CaSO₄, CaSO₄•0.5 H₂O, CaSO₄•2 H₂O, CaO, Ca(OH)₂, CaCO₃ and mixtures thereof; wherein the powder has a particle size of between 1 to 50 µm;
a carboxylic acid for forming calcium complexes when reacted with the calcium compound in the presence of water; and
a gelling agent.

2. The composition of Claim 1, wherein the gelling agent is selected from the group consisting of hydroxypropyl methyl cellulose, carboxymethyl cellulose, chitosan, collagen, gum, gelatin, and alginate, and combinations thereof.

3. The composition of Claim 1 wherein the calcium compound powder comprises tetracalcium phosphate.

4. The composition of Claim 3, wherein the tetracalcium phosphate has a calcium to phosphate molar ratio of between 1.67 to 2.

5. The composition of Claim 1 wherein the calcium compound powder comprises tricalcium phosphate.

6. The composition of Claim 5, wherein the tricalcium phosphate has a calcium to phosphate molar ratio of between 1.5 to 1.67.

7. The composition of Claim 1, wherein the calcium compound powder comprises tetracalcium phosphate and dicalcium phosphate anhydrous.

8. The composition of any of Claims 1 to 7, wherein non-toxic non-aqueous water-miscible liquid is selected from the group consisting of glycerin, propylene glycol, poly(propylene glycol), poly(ethylene glycol) and mixtures thereof.

9. The composition of any of Claims 1 to 8, wherein the carboxylic acid is selected from the group consisting of glycolic, citric, tartaric, malonic, malic, and maleic acids and combinations thereof.

10. The composition of Claim 1, wherein the mass ratio of powder to liquid is in the range of 1.5 to 1 to 3 to 1.

11. A method of preparing a paste for bone and tooth restoration, the method comprising:
(a) formulating a composition comprising a non-toxic non-aqueous water-miscible liquid; powder selected from the group consisting of monocalcium phosphate monohydrate, monocalcium phosphate anhydrous, dicalcium phosphate anhydrous, dicalcium phosphate dehydrate, octacalcium phosphate, α-tricalcium phosphate, β-tricalcium phosphate, amorphous calcium phosphate, calcium deficient hydroxyapatite, non-stoichiometric hydroxyapatite, tetracalcium phosphate, CaSO₄, CaSO₄•0.5 H₂O, CaSO₄•2 H₂O, CaO, Ca(OH)₂, CaCO₃ and mixtures thereof, wherein the powder has a particle size of between 1 to 50 µm; a carboxylic acid for forming calcium complexes with the calcium powder; and a gelling agent; the composition being formulated under substantially anhydrous conditions; and
(b) storing said composition under substantially anhydrous conditions.

12. A composition according to any of claims 1-10, for use in dental restoration, bone implants and/or bone restoration.

## Patentansprüche

1. Im Wesentlichen wasserfreie vorgemischte Materialzusammensetzung zur Zahnrestauration und für Knochenimplantate und Knochenwiederherstellung, umfassend in Kombination:
eine nicht-toxische, nicht-wässrige wassermischbare Flüssigkeit;
eine pulverisierte Calciumverbindung, ausgewählt aus der Gruppe, bestehend aus Monocalciumphosphat-Monohydrat, wasserfreiem Monocalciumphosphat, wasserfreiem Dicalciumphosphat, Dicalciumphosphat-Dihydrat, Octacalciumphosphat, α-Tricalciumphosphat, β-Tricalciumphosphat, amorphem Calciumphosphat, calciumarmem Hydroxylapatit, nichtstöchiometrischem Hydroxylapatit, Tetracalciumphosphat, CaSO₄, CaSO₄•0,5 H₂O, CaSO₄•2 H₂O, CaO, Ca(OH)₂, CaCO₃ und Mischungen davon; wobei das Pulver eine Teilchengröße zwischen 1 bis 50 µm aufweist;
eine Carbonsäure zur Bildung von Calcium-Komplexen, wenn sie mit der Calcium-Verbindung in Gegenwart von Wasser umgesetzt wird; und ein Geliermittel.

2. Zusammensetzung nach Anspruch 1, wobei das Geliermittel ausgewählt ist aus der Gruppe bestehend aus Hydroxypropylmethylcellulose, Carboxymethylcellulose, Chitosan, Collagen, Gummi, Gelatine und Alginat und Kombinationen davon.

3. Zusammensetzung nach Anspruch 1, wobei das Pulver der Calciumverbindung Tetracalciumphosphat umfasst.

4. Zusammensetzung nach Anspruch 3, wobei das Tetracalciumphosphat ein molares Verhältnis von Calcium zu Phosphat zwischen 1,67 bis 2 aufweist.

5. Zusammensetzung nach Anspruch 1, wobei das Pulver der Calciumverbindung Tricalciumphosphat umfasst.

6. Zusammensetzung nach Anspruch 5, wobei das Tricalciumphosphat ein molares Verhältnis von Calcium zu Phosphat zwischen 1,5 bis 1,67 aufweist.

7. Zusammensetzung nach Anspruch 1, wobei das Pulver der Calciumverbindung Tetracalciumphosphat und wasserfreies Dicalciumphosphat umfasst.

8. Zusammensetzung nach einem der Ansprüche 1 bis 7, wobei die nicht-toxische, nicht-wässrige wassermischbare Flüssigkeit ausgewählt ist aus der Gruppe bestehend aus Glycerin, Propylenglykol, Poly(propylenglykol), Poly(ethylenglykol) und Mischungen davon.

9. Zusammensetzung nach einem der Ansprüche 1 bis 8, wobei die Carbonsäure ausgewählt ist aus der Gruppe bestehend aus Glykolsäure, Zitronensäure, Weinsäure, Malonsäure, Äpfelsäure und Maleinsäure und Kombinationen davon.

10. Zusammensetzung nach Anspruch 1, wobei das Massenverhältnis von Pulver zu Flüssigkeit im Bereich von 1,5 zu 1 bis 3 zu 1 liegt.

11. Verfahren zur Herstellung einer Paste für Knochen- und Zahnrestauration, wobei das Verfahren umfasst:
(a) Formulierung einer Zusammensetzung, umfassend eine nicht-toxische, nicht-wässrige wassermischbare Flüssigkeit; ein Pulver, das ausgewählt ist aus der Gruppe bestehend aus Monocalciumphosphat-Monohydrat, wasserfreiem Monocalciumphosphat, wasserfreiem Dicalciumphosphat, Dicalciumphosphat-Dihydrat, Octacalciumphosphat, α-Tricalciumphosphat, β-Tricalciumphosphat, amorphem Calciumphosphat, calciumarmem Hydroxylapatit, nichtstöchiometrischem Hydroxylapatit, Tetracalciumphosphat, CaSO₄, CaSO₄•0,5 H₂O, CaSO₄•2 H₂O, CaO, Ca(OH)₂, CaCO₃ und Mischungen davon; wobei das Pulver eine Teilchengröße zwischen 1 bis 50 µm aufweist; eine Carbonsäure zur Bildung von Calciumkomplexen mit dem Calciumpulver; und ein Geliermittel; wobei die Zusammensetzung im Wesentlichen unter wasserfreien Bedingungen formuliert wird; und
(b) Lagern der Zusammensetzung unter im Wesentlichen wasserfreien Bedingungen.

12. Zusammensetzung nach einem der Ansprüche 1 - 10 zur Verwendung für die Zahnrestauration, für Knochenimplantate und/oder Knochenwiederherstellung.

## Revendications

1. Composition pré-mélangée essentiellement exempte d'eau d'une matière pour la restauration dentaire, les implants osseux et la restauration osseuse, comprenant en combinaison :
un liquide non toxique non aqueux miscible à l'eau ;
un composé de calcium pulvérulent choisi dans le groupe constitué du phosphate monocalcique monohydraté, du phosphate monocalcique anhydre, du phosphate dicalcique anhydre, du phosphate dicalcique déshydraté, du phosphate octocalcique, du phosphate α-tricalcique, du phosphate β-tricalcique, du phosphate de calcium amorphe, de l'hydroxyapatite pauvre en calcium, de l'hydroxyapatite non stoechiométrique, du phosphate tétracalcique, du CaSO₄, du CaSO₄.0,5 H₂O, du CaSO₄.2 H₂O, du CaO, du Ca(OH)₂, du CaCO₃ et de leurs mélanges ; dans laquelle la poudre a une taille particulaire entre 1 et 50 µm ;
un acide carboxylique pour former des complexes de calcium lorsqu'il réagit avec le composé de calcium en présence d'eau ; et
un agent gélifiant.

2. Composition selon la revendication 1, dans laquelle l'agent gélifiant est choisi dans le groupe constitué de l'hydroxypropylméthyl-cellulose, de la carboxyméthylcellulose, du chitosane, du collagène, de la gomme, de la gélatine et d'un alginate, et de leurs combinaisons.

3. Composition selon la revendication 1, dans laquelle la poudre de composé de calcium comprend du phosphate tétracalcique.

4. Composition selon la revendication 3, dans laquelle le phosphate tétracalcique présente un rapport molaire du calcium au phosphate entre 1,67 et 2.

5. Composition selon la revendication 1, dans laquelle la poudre de composé de calcium comprend du phosphate tricalcique.

6. Composition selon la revendication 5, dans laquelle le phosphate tricalcique présente un rapport molaire du calcium au phosphate entre 1,5 et 1,67.

7. Composition selon la revendication 1, dans laquelle la poudre de composé de calcium comprend du phosphate tétracalcique et du phosphate dicalcique anhydre.

8. Composition selon l'une quelconque des revendications 1 à 7, dans laquelle le liquide non toxique non aqueux miscible à l'eau est choisi dans le groupe constitué de la glycérine, du propylèneglycol, du poly(propylèneglycol), du poly(éthylèneglycol) et de leurs mélanges.

9. Composition selon l'une quelconque des revendications 1 à 8, dans laquelle l'acide carboxylique est choisi dans le groupe constitué des acides glycolique, citrique, tartrique, malonique, malique et maléique et de leurs combinaisons.

10. Composition selon la revendication 1, dans laquelle le rapport massique de la poudre au liquide se situe dans la plage de 1,5 à 1 à 3 à 1.

11. Procédé de préparation d'une pâte pour la restauration des os et des dents, le procédé comprenant les étapes consistant à :
(a) formuler une composition comprenant un liquide non toxique non aqueux miscible à l'eau ; une poudre choisie dans le groupe constitué du phosphate monocalcique monohydraté, du phosphate monocalcique anhydre, du phosphate dicalcique anhydre, du phosphate dicalcique déshydraté, du phosphate octocalcique, du phosphate α-tricalcique, du phosphate β-tricalcique, du phosphate de calcium amorphe, de l'hydroxyapatite pauvre en calcium, de l'hydroxyapatite non stoechiométrique, du phosphate tétracalcique, du CaSO₄, du CaSO₄.0,5 H₂O, du CaSO₄.2 H₂O, du CaO, du Ca(OH)₂, du CaCO₃ et de leurs mélanges, dans lequel la poudre a une taille particulaire entre 1 et 50 µm ; un acide carboxylique pour former des complexes de calcium avec la poudre de calcium ; et un agent gélifiant ; la composition étant formulée dans des conditions sensiblement anhydres ; et
(b) stocker ladite composition dans des conditions sensiblement anhydres.

12. Composition selon l'une quelconque des revendications 1 à 10 pour utilisation en restauration dentaire, pour les implants osseux et/ou en restauration osseuse.
